# EUROPEAN PATENT SPECIFICATION

(11) **EP 3 833 666 B1**
(45) Date of publication and mention of the grant of the patent: **02.11.2022**
(21) Application number: 19779557.8
(22) Date of filing: 02.08.2019
(51) Int. Cl.: C07D 473/06

(54) **PROCESSES FOR THE PREPARATION OF BOC-LINAGLIPTIN**
VERFAHREN ZUR HERSTELLUNG VON BOC-LINAGLIPITIN
PROCÉDÉS POUR LA PRÉPARATION DE BOC-LINAGLIPTINE

(30) Priority: 06.08.2018 HU 1800278
(43) Date of publication of application: 16.06.2021
(73) Proprietor: Richter Gedeon Nyrt., 1103 Budapest (HU)
(72) Inventor: PINTÉR, Gábor, 1224 Budapest (HU); MOLNÁR, Katalin, 1116 Budapest (HU); SEBÕK, Ferenc, 2217 Gomba (HU)
(74) Representative: Kutzenberger Wolff & Partner
(86) International application number: PCT/IB2019/056592
(87) International publication number: WO 2020/031040

(56) References cited:
- CN-A- 110 240 599
- US-A1- 2004 138 215
- Anon: "Preparation of ( (R)-8-(3-amino-piperidin-1-yl)-7-(but-2-yn yl)-3-methyl-1- (4-methyl-quinazolin-2-ylmethyl)-3,7-dihyd ro-purine-2,6-dione", ip.com, 13 August 2014 (2014-08-13), XP055633967, Retrieved from the Internet: URL:https://ip.com/IPCOM/000238270 [retrieved on 2019-10-21]

## Description

### FIELD OF THE INVENTION

The present invention relates to novel processes for the synthesis of a xanthine-based pharmaceutically active ingredient, i.e., Linagliptin, and intermediates thereof. In particular, the present invention relates to a process for the preparation of Quinazolinyl-xanthine of Formula 4 and to a process for the preparation of BOC-Linagliptin of Formula 2.

The compounds according to Formulae 3 and 5 are not claimed as such. Further, processes for the preparation of the compounds according to Formulae 3 and 5 are not claimed. References to these compounds essentially serve information purposes.

### BACKGROUND OF THE INVENTION

Linagliptin is a compound of Formula 1 chemically known as 8-[(3R)-3-amino-1-piperidinyl]-7-(2-butyn-1-yl)-3-methyl-1-[(4-methyl-2-quinazolinyl)methyl]-3,7-dihydro-1H-purine-2,6-dione.

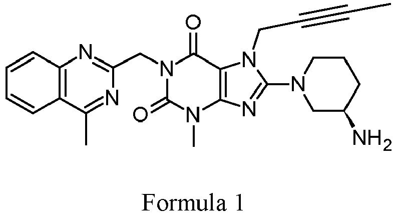

Linagliptin is used for the treatment of type-II diabetes mellitus and marketed in the United States under the trade name Trajenta and in combination with metformin hydrochloride under the trade name Jentadueto.

There are several methods describing the synthesis of Linagliptin known in literature.

WO 2004/018468 and US 7,407,955 disclose the following process for the preparation of Linagliptin:

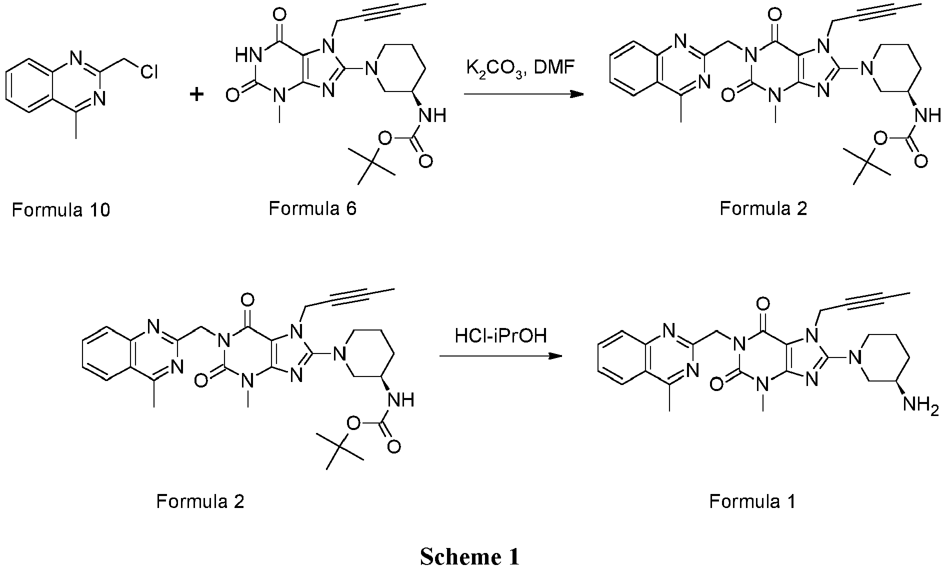

Impurities forming in this process are difficult to remove, especially on industrial scale and are attributed to the protecting group used.

WO 2006/048427 and US 7,820,815 describe the following process for the synthesis of Linagliptin:

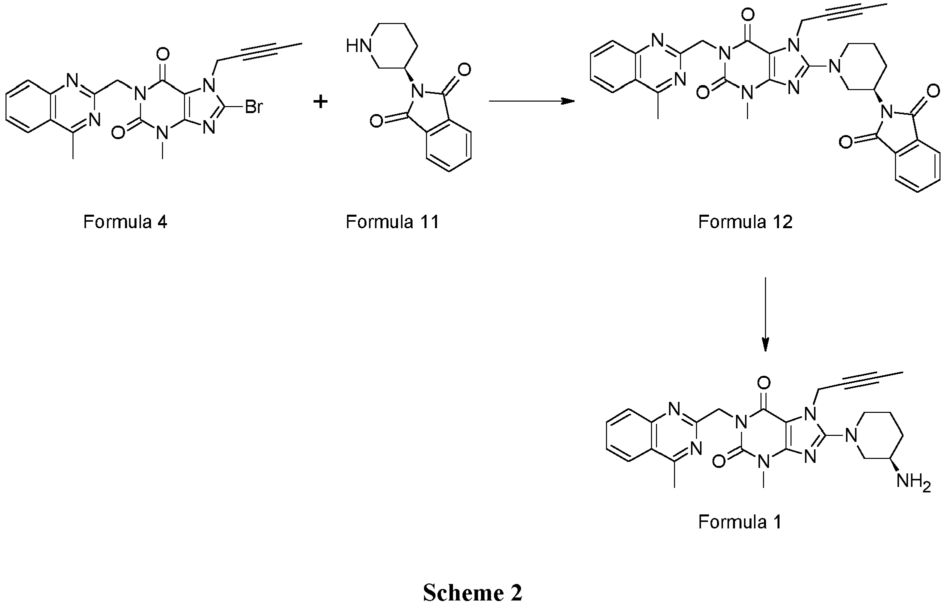

US 7,820,815 also discloses the preparation of 2-chloromethyl-4-methylquinazoline in the following way:

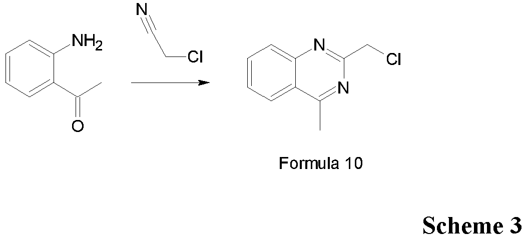

The reference WO 2015/067539 describes a method for the synthesis of quinazolinyl-xanthine (Formula 4): 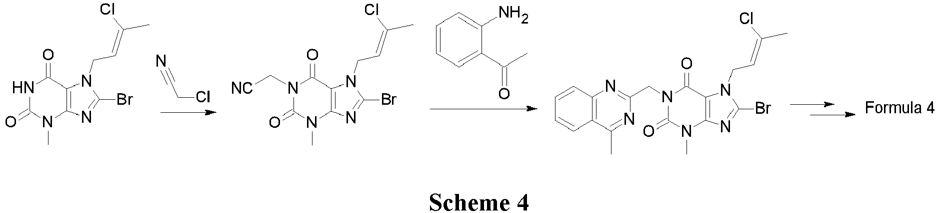 and that of Linagliptin (Formula 1):

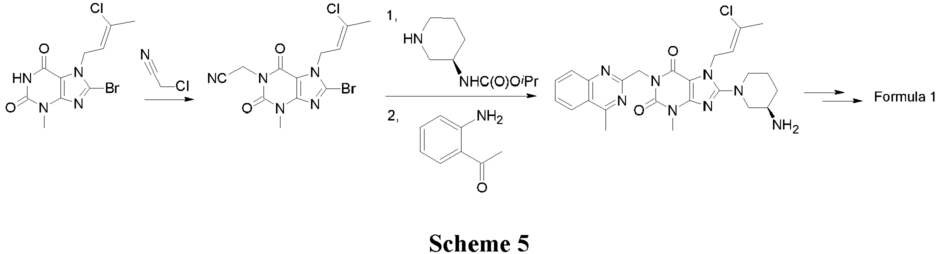

### SUMMARY OF THE INVENTION

Our invention is summarized in Scheme 6.

This invention is a new, industrially applicable process for the preparation of BOC-Linagliptin of Formula 2, in new synthetic routes via intermediates (Formula 5 and Formula 3). These intermediates enable the formation of the quinazoline part of Linagliptin on the xanthine scaffold in a ring-closing reaction.

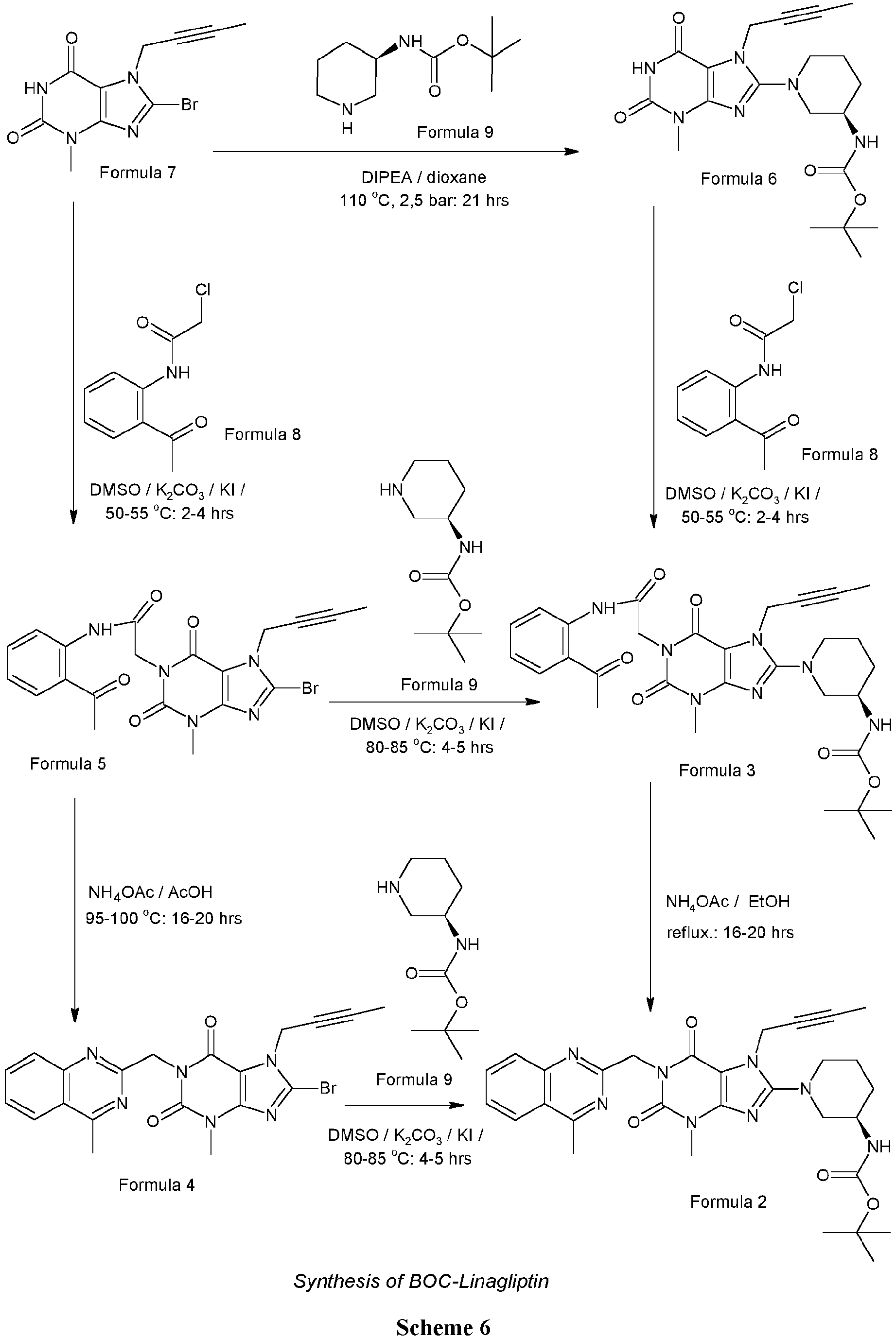

### DETAILED DESCRIPTION OF THE INVENTION

According to Scheme 6, there are three different routes available for preparation of BOC-Linagliptin that are different embodiments of this invention.

In one possible route reacting Butynyl-Br-Me-xanthine of Formula 7 with Acetylphenyl-chloroacetamide of Formula 8, Acetylphenylamino-But-Br-Me-xanthine of Formula 5 is formed **(*Example 2*)***.* The alkylation of Butynyl-Br-Me-xanthine can be carried out in dipolar aprotic solvents such as dimethylformamide (DMF), dimethylacetamide (DMAA), *N*-methyl-2-pyrrolidone (NMP), dimethyl sulfoxide (DMSO) using inorganic bases like sodium, potassium, cesium carbonate or hydrogen carbonate. Sodium or potassium iodide in catalytic amount accelerate the reaction. The reaction advantageously takes place in DMSO solution in the presence of potassium iodide and potassium carbonate. The temperature of this reaction is in the range of 40-90 °C, advantageously in 50-55 °C. The formed intermediate is transformed into Quinazolinyl-xanthine of Formula 4 at temperature between 60 and 120 °C in a ring-closing reaction **(*Example 3*)***.* Ammonia, ammonium acetate, ammonium formate, ammonium chloride and the like can be used as nitrogen- source for ring-closure. The solvent was selected from among alcohols (like methanol, ethanol or 2-propanol), acetic acid, toluene and the like. To accomplish this reaction, the mixture is stirred at 95-100 °C for 16-20 hrs, preferably in acetic acid. Quinazolinyl-xanthine of Formula 4 is reacted with Boc-aminopiperidine of Formula 9 resulting the formation of Boc-Linagliptin of Formula 2 **(*Example* 7)**. The reaction can be carried out in different polar aprotic solvents (e.g. DMF, DMAA, NMP, DMSO) in the presence of base (e.g. sodium, potassium, cesium carbonate or bicarbonate) and sodium or potassium iodide as catalyst. This *N*-arylation requires higher temperature (60-120 °C) than alkylation of Butynyl-Br-Me-xanthine. The reaction preferably takes place in DMSO in the presence of potassium carbonate and potassium iodide at 80-85 °C.

In the next possible route, Butynyl-Br-Me-xanthine of Formula 7 can be reacted with Acetylphenyl-chloroacetamide of Formula 8 while Acetylphenylamino-But-Br-Me-xanthine of Formula 5 is formed (for the reaction conditions see ***Example 2*)***.* This intermediate can be reacted with Boc-aminopiperidine of Formula 9 to prepare Acetylphenylamino-piperidinyl-xanthine of Formula 3 **(*Example 5*)*****.*** The applied conditions could be the same as in the reaction of Quinazolinyl-xanthine of Formula 4 with Boc-aminopiperidine of Formula 9. The reaction advantageously can be carried out in DMSO at 80-85 °C using potassium carbonate as base and potassium iodide as catalyst. This latter compound can be converted into the aimed Boc-linagliptin of Formula 2 **(*Example* 6)** in a ring-closing reaction. Ammonia, ammonium acetate, ammonium formate, ammonium chloride and the like can be used as nitrogen- source for ring-closure. The solvent was selected from among alcohols (like methanol, ethanol or 2-propanol) or toluene at temperature between 60 and 120 °C. Acetic acid is not recommended because of the acid-sensitive Boc protecting group.

According to Scheme 6, in a third route, Butynyl-Br-Me-xanthine of Formula 7 can be reacted with Boc-aminopiperidine of Formula 9 to get Piperidinyl-xanthine of Formula 6. The reaction can be accomplished in solvents such as DMSO, DMAA, NMP, acetonitrile, 1,4-dioxane in the presence of inorganic (e.g. sodium, potassium, cesium carbonate or bicarbonate) or organic base (e.g. triethylamine or *N,N-*diisopropylethylamine) at temperature of 60 to 140 °C. The reaction advantageously takes place in Dioxane in the presence of N,N-Diisopropylethylamine (DIPEA) at 110 °C. Reacting Piperidinyl-xanthine of Formula 6 with Acetylphenyl-chloroacetamide of Formula 8, the compound of Formula 3 **(*Example 4*)** is prepared. This latter compound can be converted into the aimed Boc-Linagliptin of Formula 2 in a ring-closing reaction **(*Example 6*)** (for the reaction conditions see ***Example 3*)***.*

### EXAMPLES

### Example 1: Synthesis of Acetylphenyl-chloroacetamide (Formula 8)

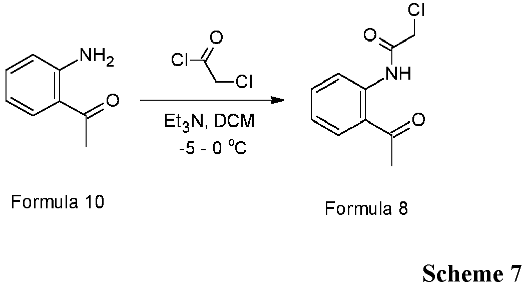

To a solution of 2'-aminoacetophenone (9.0 mL, 10.0 g, 74 mmol; 1 eq) in dry dichloromethane (80 mL) was added triethylamine (15.5 mL, 11.23 g, 111 mmol; 1.5 eq) under nitrogen atmosphere. To the reaction mixture the solution of chloroacetyl chloride (8.2 mL, 11.7 g, 104 mmol; 1.4 eq) in DCM (20 mL) was slowly added at -5-0 °C, then the reaction mixture was stirred under such conditions for 2 hrs. The reaction was monitored by TLC (silica, hexane:ethyl-acetate=7:3). The reaction mixture was diluted with DCM (150 mL) and washed with aq. NaHCO₃ (5%, 1x60 mL), hydrochloric acid (10%, 2x40 mL) and brine (1x40 mL), respectively. The organic phase was dried and evaporated under reduced pressure at 30-35 °C. The residue was recrystallized from 2-propanol and dried under vacuum at 50 °C to get acetylphenyl-chloroacetamide as a beige powder (12.7 g, HPLC: 96.7%).

### Example 2: Synthesis of Acetylphenylamino-But-Br-Me-xanthine (Formula 5)

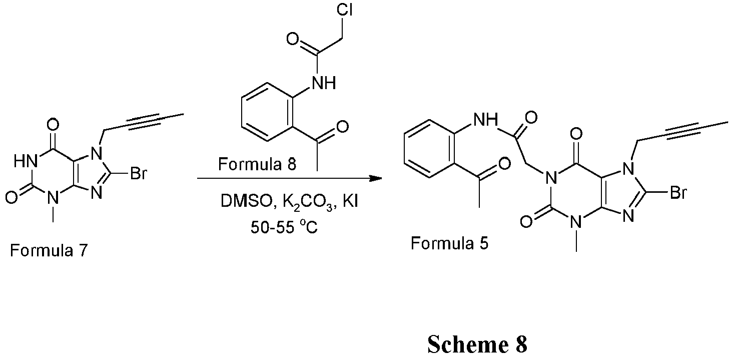

To a solution of butynyl-Br-Me-xanthine [Formula 7] (5.0 g, 16.9 mmol, 1 eq) in DMSO (65 mL) acetylphenyl-chloroacetamide [Formula 8] (3.55 g, 16.9 mmol, 1 eq), potassium iodide (0.28 g, 1.69 mmol, 0.1 eq) and powdered potassium carbonate (2.55 g, 18.6 mmol, 1.1 eq) were added under nitrogen atmosphere. The reaction mixture was heated to 50-55 °C and stirred for 2-4 hrs. (The reaction was monitored by TLC (silica; DCM:MeOH=95:5).) The reaction mixture was cooled down to room temperature and water was added dropwise. The suspension was stirred for 1 hr, then the precipitate was filtered, washed with water and dried in vacuum at 50 °C to get the product as a pale yellow powder (7.4 g, HPLC: 97.4%).

### Example 3: Synthesis of Quinazolinyl-xanthine (Formula 4)

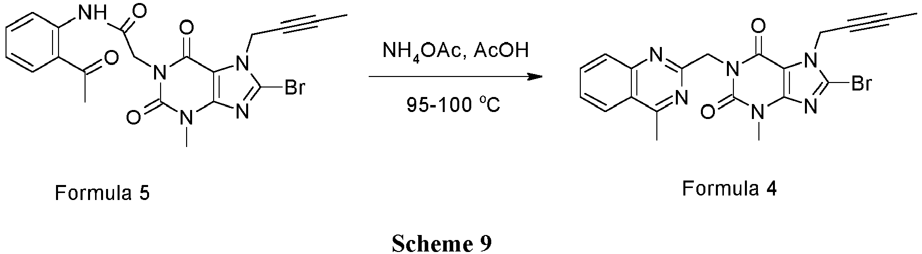

To a suspension of acetylphenylamino-But-Br-Me-xanthine [Formula 5] (5.0 g, 10.6 mmol, 1 eq) in acetic acid (50 mL) ammonium acetate (8.0 g, 106 mmol, 10 eq) was added under nitrogen atmosphere and the reaction mixture was stirred at 95-100 °C for 16-20 hrs. The reaction was monitored by TLC (silica; hexane:ethyl acetate = 6:4). The reaction mixture was cooled down to room temperature and water (150 mL) was added dropwise during vigorous stirring (the temperature was kept below 25 °C). The reaction mixture was stirred at room temperature for 1 hour. The precipitate was filtered, washed with water (2×20 mL) and dried at 50 °C in vacuum to obtain the product as a yellow powder (4.4 g, HPLC: 95.2%), which was purified by suspending in 2-propanol (off-white powder, yield: 95%, HPLC: 97%).

### Example 4: Synthesis of Acetylphenylamino-piperidinyl-xanthine (Formula 3)

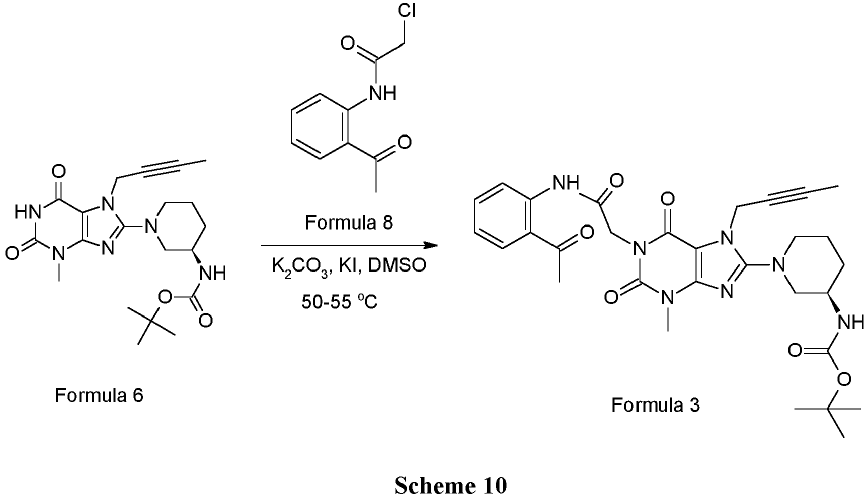

To a suspension of piperidinyl-xanthine [Formula 6] (5.0 g, 12.0 mmol, 1 eq) in DMSO (75 mL) acetylphenyl-chloroacetamide [Formula 8] (2.54 g, 12 mmol, 1 eq), powdered K₂CO₃ (1.82 g, 13.2 mmol, 1.1 eq) and KI (0.2 g, 10 mol%) were added under nitrogen atmosphere and the reaction mixture was stirred at 50-55 °C for 4-6 hrs. The reaction was monitored by TLC (silica; DCM:MeOH=95:5). The reaction mixture was cooled down to room temperature and water (300 mL) was added dropwise. The resulted suspension was stirred for one hour. The precipitate was filtered, washed with water (2×25 mL) and dried under vacuum at 50 °C to get the product as a beige powder (6,98 g, HPLC: 88,6%).

### Example 5: Synthesis of Acetylphenylamino-piperidinyl-xanthine (Formula 3)

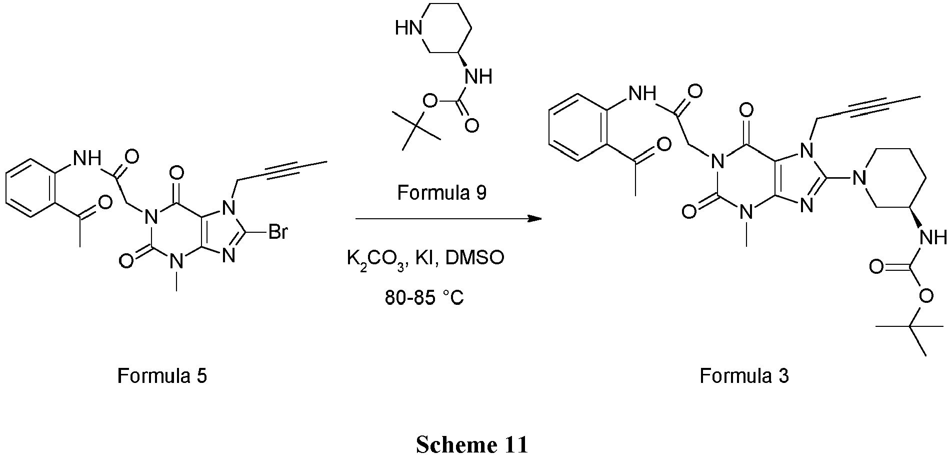

To a suspension of acetylphenylamino-But-Br-Me-xanthine [Formula 5] (1.0 g, 2.12 mmol, 1 eq) in DMSO (10 mL) Boc-aminopiperidine [Formula 9] (0.51 g, 2.54 mmol, 1.2 eq), potassium carbonate (0.44 g, 3.18 mmol, 1.5 eq) and potassium iodide (50 mg, 10 w/w%) were added in inert atmosphere. The temperature was raised to 80-85 °C and the reaction mixture was stirred at this temperature for 4-5 hrs. The reaction was followed by TLC (silica; DCM:MeOH = 95:5). After completion of the reaction reaction mixture was cooled to room temperature, water (40 mL) was added dropwise and the reaction mixture was stirred at 20-25 °C for half hour. The precipitate was filtered, washed with water and dried at 50 °C in vacuum to get titled compound as a pale brown powder. (1.1 g, HPLC: 92.3%)

### Example 6: Synthesis of BOC-Linagliptin (Formula 2)

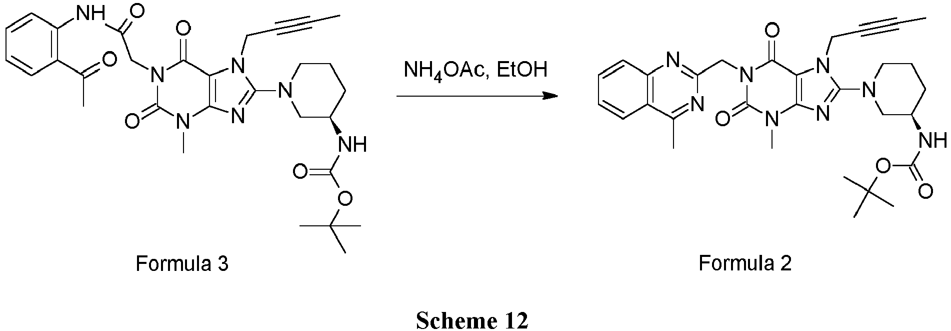

To a suspension of acetylphenylamino-piperidinyl-xanthine [Formula 3] (1.0 g, 1.69 mmol, 1 eq) in abs. ethanol (20 mL) ammonium acetate (1.3 g, 16.9 mmol, 10 eq) was added under nitrogen atmosphere and the reaction mixture was stirred at reflux temperature for 16-20 hrs. The reaction was monitored by TLC (silica; DCM:MeOH = 95:5). The reaction mixture was cooled down to room temperature and water (60 mL) was added dropwise during vigorous stirring (the temperature was kept below 25 °C). The reaction mixture was stirred at room temperature for 1 hour. The precipitate was filtered, washed with water (2×5 mL) and dried at 50 °C in vacuum to obtain the product as a yellowish brown powder (∼100%, HPLC: 90.2%).

### Example 7: Synthesis of Boc-Linagliptin (Formula 2)

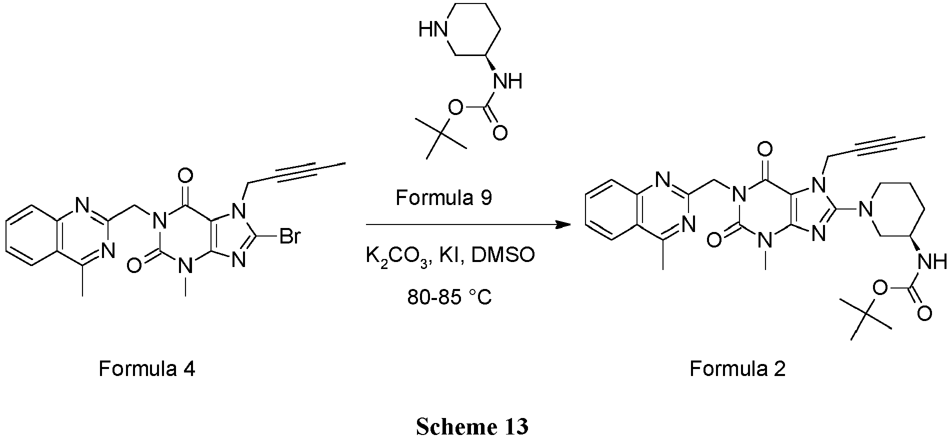

To a suspension of quinazolinyl xanthine [Formula 4] (1.0 g, 2.21 mmol, 1 eq) in DMSO (10 mL) BOC-aminopiperidine [Formula 9] (0.53 g, 2.65 mmol, 1.2 eq), potassium carbonate (0.46 g, 3.32 mmol, 1.5 eq) and potassium iodide (50 mg, 10 w/w%) were added under nitrogen atmosphere. The reaction mixture was heated to 80-85 °C and stirred for 4-5 hrs. The reaction was followed by TLC (silica; DCM:MeOH = 95:5). The reaction mixture was cooled down to room temperature, water (40 mL) was added dropwise and the reaction mixture was stirred at 20-25 °C for half hour. The precipitate was filtered, washed with water and dried under vacuum at 50 °C to get title compound [Formula 2] as a pale yellow powder (1.2 g, HPLC: 96.7%).

## Claims

1. The process for the preparation of Quinazolinyl-xanthine of Formula 4 comprising reacting the compound Acetylphenylamino-But-Br-Me-xanthine of Formula 5 in an alcohol or in an aromatic hydrocarbon solvent using ammonia or an ammonium salt as N-source according to Scheme 9:

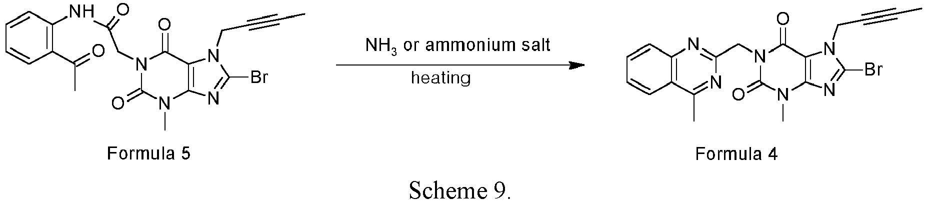

2. The process for the preparation of BOC-Linagliptin of Formula 2 comprising reacting the compound Acetylphenylamino-piperidinyl-xanthine of Formula 3 in an alcohol or in an aromatic hydrocarbon solvent using ammonia or an ammonium salt as N-source according to Scheme 12:

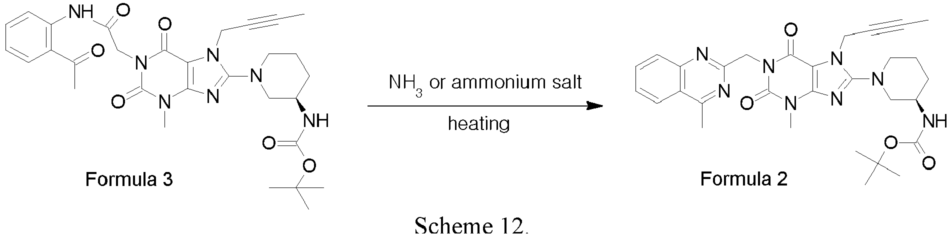

3. The process according to claim 1 and 2 wherein as solvent ethanol and as N-source ammonium acetate are used and the reaction is carried out at the reflux temperature.

## Patentansprüche

1. Verfahren zur Herstellung von Chinazolinyl-Xanthin der **Formel 4,** umfassend die Umsetzung der Verbindung Acetylphenylamino-But-Br-Me-Xanthin der **Formel 5** in einem Alkohol oder in einem aromatischen Kohlenwasserstofflösungsmittel unter Verwendung von Ammoniak oder einem Ammoniumsalz als N-Quelle gemäß Schema 9:

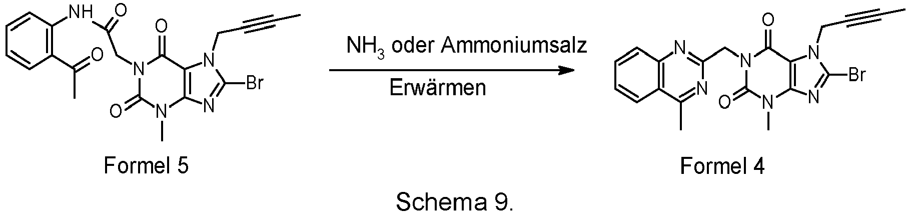

2. Das Verfahren zur Herstellung von BOC-Linagliptin der **Formel 2**, umfassend die Umsetzung der Verbindung Acetylphenylamino-piperidinyl-xanthin der **Formel 3** in einem Alkohol oder in einem aromatischen Kohlenwasserstofflösungsmittel unter Verwendung von Ammoniak oder einem Ammoniumsalz als N-Quelle gemäß Schema 12:

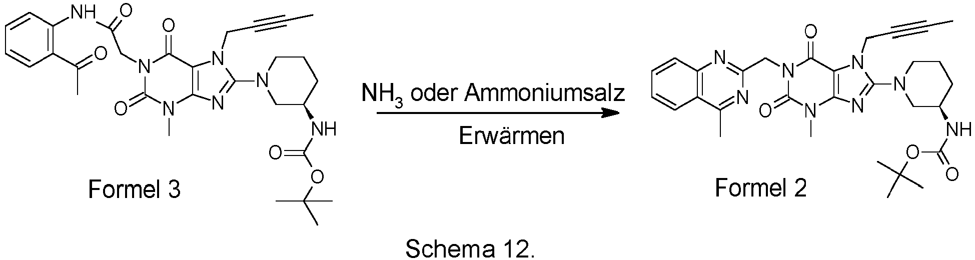

3. Das Verfahren gemäß Anspruch 1 und 2, wobei als Lösungsmittel Ethanol und als N-Quelle Ammoniumacetat verwendet werden und die Reaktion bei Rückflusstemperatur durchgeführt wird.

## Revendications

1. Procédé de préparation de quinazolinyl-xanthine de formule 4 comprenant la réaction du composé acétylphénylamino-But-Br-Me-xanthine de formule 5 dans un alcool ou dans un solvant hydrocarbure aromatique en utilisant de l'ammoniac ou un sel d'ammonium en tant que source de N selon le schéma 9 :

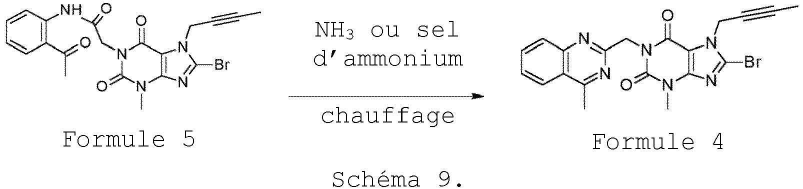

2. Procédé de préparation de BOC-linagliptine de formule 2 comprenant la réaction du composé acétylphénylamino-pipéridinyl-xanthine de formule 3 dans un alcool ou dans un solvant hydrocarbure aromatique en utilisant de l'ammoniac ou un sel d'ammonium en tant que source de N selon le schéma 12 :

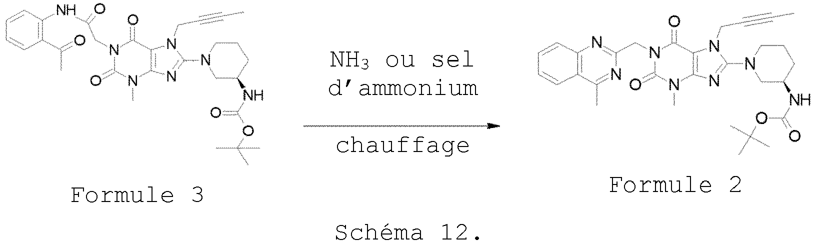

3. Procédé selon les revendications 1 et 2 dans lequel, en tant que solvant, l'éthanol et, en tant que source de N, l'acétate d'ammonium sont utilisés et la réaction est conduite à la température de reflux.
